(19) Europäisches Patentamt
European Patent Office
Office européen des brevets

(11) Publication number: **0 348 664 A2**

(12) # EUROPEAN PATENT APPLICATION

(21) Application number: 89109229.8

(51) Int. Cl.4: A61K 31/22

(22) Date of filing: 23.05.89

The title of the invention has been amended (Guidelines for Examination in the EPO, A-III, 7.3).

(30) Priority: 26.05.88 JP 129607/88

(43) Date of publication of application: 03.01.90 Bulletin 90/01

(84) Designated Contracting States: CH DE FR GB IT LI NL SE

(71) Applicant: OTSUKA PHARMACEUTICAL FACTORY, INC.
115, Aza Kuguhara Tateiwa Muya-cho
Naruto-shi Tokushima 772(JP)

Applicant: Nagayama, Masayoshi
1-15, Bandaihigashi-1-chome
Sumiyoshi-ku Osaka(JP)

(72) Inventor: Umeyama, Kaoru
3-30, Hibarigaoka-1-chome
Takarazuka-shi(JP)
Inventor: Nagayama, Masayoshi
1-15, Bandaihigashi-1-chome
Sumiyoshi-ku Osaka(JP)
Inventor: Nishiguchi, Yukio
Inperiaru Senriaobaoka 1009 12-38, Aobaokaminami
Suita-shi(JP)
Inventor: Okuno, Masahiro
7-7, Kannocho
Nishinomiya-shi(JP)
Inventor: Tanaka, Shunji
5-1, Takeshirodai-3-cho
Sakai-shi(JP)
Inventor: Ikehara, Teruyuki
490, Takenouchi Taimacho
Kitakatsuragi-gun Nara-ken(JP)
Inventor: Iwamoto, Tetsuji
20, Aza Shimai Tateiwa
Muyacho Naruto-shi(JP)

(74) Representative: Hansen, Bernd, Dr.rer.nat. et al
Hoffmann, Eitle & Partner Patentanwälte
Arabellastrasse 4 Postfach 81 04 20
D-8000 München 81(DE)

(54) **Pharmaceutical compositions containing monoacetoacetins for treating tumours.**

(57) A pharmaceutical composition for treating tumor containing, as the active ingredient, any one or both of monoacetoacetins of the formulas (1) and (2). Said monoacetoacetins are known compounds and each one of them composed of glycerin and acetoacetic acid in it structural formula. However, up to date, there have not been known that these monoacetoacetins possess anti-tumor activities.

EP 0 348 664 A2

# PHARMACEUTICAL COMPOSITION FOR TREATING TUMOR

## TECHNICAL FIELD

The present invention relates to a pharmaceutical composition for treating tumor and more particularly it relates to a pharmaceutical composition for treating tumor containing monoacetoacetin as the active ingredient.

## PRIOR ART AND PROBLEMS THEREIN

There have heretofore been conducted various investigations on the relationship between malignant tumor and nutrition. Most of the investigations indicate that feeding causes an enlargement of tumor and that the worsening of nutritive conditions inhibits the multiplication of tumor. That is, they indicate that sufficient feeding to a tumor bearing animal results not only in improvement of the general conditions and the bio-reactions such as immunological ability but also in multiplication of the tumor. A method for feeding to a tumor bearing animal is required to have effects contrary to each other, i.e., maintenance of the nutritive conditions by feeding, and inhibition of the tumor. Such an ideal feeding method and a pharmaceutical composition for practicing it have not yet been developed.

On the other hand, ketone bodies which have been known as physiological energy sources since early times are not easily metabolized in cancerous cells [Rofe, A.M. et al., Biochem. J., 233, 485-491 (1986)]. Among the ketone bodies, butyrates are known to promote the differentiation of cancerous cells [Motoo Hozumi "Cancer and Chemotherapy" 14, 1349-1357 (1987)]. A remedy for cancer in which the multiplication of cancerous cells is suppressed by the promotion of differentiation of cancerous cells to cause reduction of metastasis and the like is biginning to be noted as so-called differentiation therapy. From such a viewpoint, the present inventors also have conducted various investigation of remedies for cancer, anti-tumor agents used therein, etc. In the course of the investigations, the present inventors noted monoacetoacetin which has recently been reported as an effective energy source for a living body under the stress of an external wound or an operation [Nippon Shokaki Geka Gakkai Shi, 20 (5), 1087-1092 (1987)]. The present inventors carried out an experiment in which this compound was intravenously administered to tumor bearing rats, and we consequently found the following facts. Said compound also functions in the tumor bearing animal as an energy source which keeps the nutritive conditions good as described in the above report. In addition, surprisingly, said compound acts on tumor cells as an anti-tumor agent capable of inhibiting their multiplication and is effective in curing cancer. Utilization of said compound permits establishment of an ideal method for feeding to a tumor bearing animal. The present invention has been accomplished on the basis of the above finding.

## MEANS FOR SOLVING THE PROBLEMS

That is, according to the present invention, a pharmaceutical composition for treating tumor containing monoacetacetin as active ingredient is provided.

Monoacetoacetin used as active ingredient in the pharmaceutical composition for treating tumor of the present invention is a well known compound composed of glycerin and acetoacetic acid in its structural formula and can be produced, for example, by the method described in a reference [see Journal of Pharmaceutical Sciencies 72, No. 3, pp. 280, (1983), or Organic Synthesis 5, 28-29, (1962)]. Said compound has two position isomers represented by the formulas shown below, and both isomers can be similarly utilized in the present invention and can similarly bring about the desired effects of the present invention. Needless to say, combined use of the two isomers is also possible.

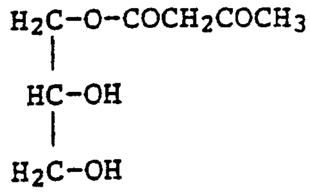

(1)

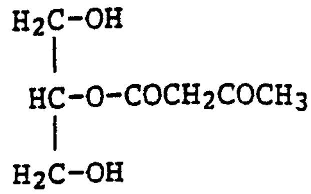

(2)

The pharmaceutical composition for treating tumor of the present invention contains any one or both of the above monoacetoacetins as the active ingredient. The monoacetoacetin(s) is formulates into a conventional pharmaceutical form in combination with a suitable diluent, other additives, etc. Said composition is administered by an administration route suitable for the pharmaceutical form. Typical examples of the pharmaceutical form are infusion preparations and liquid form preparations which are suitable for administration intravenous such as peripheral veins and central veins and enteral (tube and oral feeding) administration, respectively, like amino acid infusions and electrolyte infusions.

The formulation into the above various pharmaceutical forms can be carried out according to a method conventionally used in the art. As to the diluent, additives and the like used for the formulation, there can be used various diluents, additives and the like which are conventionally used for preparing well-known preparations having the same forms as those of the various preparations.

More specifically, in the case of formulation into an infusion, the pharmaceutical composition of treating tumor of this invention is prepared by diluting an effective amount of the aforesaid compound as an active ingredient in a suitable diluent such as water for injection or the like, adding additives such as a stabilizer and an agent for adjusting pH if necessary, and sterilizing the resulting liquid such as aqueous solution by conventional sterilizing filtration or the like. The pH of the thus prepared pharmaceutical composition of the present invention in infusion form is preferably pH 3.0-8.0, or more preferably pH 5.0-7.5. The concentration of the compound as an active ingredient in said infusion is usually about 3-25% (W/V%, hereinafter the same applied), preferably about 5-20%, or more preferably about 10%.

The aforesaid pharmaceutical composition of the present invention in infusion form can bring about excellent feeding effect and carcinostatic effect or anti-tumor effect which are desired effects of the present invention, most preferably when it is administered through central vein in the form of an aseptic aqueous solution, namely, when it is used in the so-called total parenteral nutrition (TPN).

As to the dose of the pharmaceutical composition containing any one or both of the aforesaid monoacetoacetins as an active ingredient for treating tumor of the present invention in infusion form, a standard dose is usually about 100-4000 ml per adult a day and the dose can be properly increased or decreased depending on the pathosis, nutritive conditions, age and body weight of a patient to whom said pharmaceutical composition is to be administered, other drugs can be used together with said pharmaceutical composition, etc.

On the other hand, the pharmaceutical composition for treating tumor of the present invention can be administered also orally or through a canal in the form of the aforesaid infusion or a liquid form preparation other than the aforesaid infusion. Also in the case of such administration, said pharmaceutical composition can bring about excellent feeding effect and cancerocidal effect or anti-tumor effect which are desired effects of the present invention. The liquid form preparation can be prepared by a conventional method, for example, by using the compound as an active ingredient together with conventional diluents such as distilled water and if necessary, other additives. As to the additives usable in preparing the aforesaid liquid form preparation, there can be exemplified, conventional dissolution assistants, buffers, anodynes, preservatives, coloring agents, perfumes, flavoring materials, and sweetening agents. Suitable amounts of a sodium chloride solution, a sucrose solution, a glycerin solution, etc. can also be included in the aforesaid liquid form preparation in order to make this preparation isotonic. The dose of the liquid form preparation thus prepared may be substantially the same as that of the aforesaid infusion for intravenous administration. Needless to say, it can properly be increased or decreased depending on the pathosis, nutritive conditions, age and body weight of a patient to whom the liquid form preparation is to be administered, other drugs

used together with the liquid form preparation, etc.

In the pharmaceutical composition for treating tumor of this invention, protein nutrition sources such as amino acids can properly be blended with monoacetoacetin(s) as an essential active ingredient of the pharmaceutical composition. In addition to the protein nutrition sources, there can be blended electrolytes and other energy sources which are usually used in nutrient infusions, for example, sugars such as glucose, fructose, xylitol, sorbitol, maltose and the like, and polyhydric alcohols such as glycerol and the like. Furthermore, various vitamins, lipids, trace elements and the like can optionally be added into the pharmaceutical composition for treating tumor of the present invention. The above various ingredients can be previously added into the pharmaceutical composition for treating tumor containing monoacetoacetin of the present invention to prepare one pharmaceutical composition, or they can be mixed with said pharmaceutical composition of the present invention at the time of use of the composition.

Furthermore, the pharmaceutical composition for treating tumor of the present invention, irrespective of its pharmaceutical form and administration rate, can be used in combination with various anti-cancer agents which are known as chemotherapeutics, or with radiotherapy. In this case, since the active ingredient of this invention can bring about an excellent anti-tumor effect, it can heighten the effect of the anti- cancer agent used together therewith to obtain a synergistic effect. Therefore, a sufficient tumor-treating effect can be obtained even when the amount of the anti-cancer agent co-used is considerably smaller than its usual dose. Accordingly, the adverse side effects of the anti-cancer agents can be reduced. On the other hand, since the active ingredient of the present invention is an excellent energy source, the aforesaid anti-cancer agent to be co-used can be administered in a large amount. Such chemotherapeutic agents include, for example, 5-fluorouracil (5-FU, mfd. by Kyowa Hakko Kogyo Co., Ltd.), mitomycin (mitomycin C manufactured by Kyowa Hakko Kogyo Co., Ltd.), Tegafur (Futraful, manufactured by Taiho Pharmaceutical Company Ltd.), Cyclophosphamide (Endoxaan, mfd. by Shionogi & Co., Ltd.), Chromomycin A3 (Toyomycin, mfd. by Takeda Chemical Industries, Ltd.).

## EXAMPLES

The following examples (preparation examples) of the pharmaceutical composition for treating tumor of the present invention and the pharmacological test examples next thereto illustrate the present invention in more detail without limiting the scope of the invention. Monoacetoacetin used in the examples was prepared by the method described in Organic Synthesis 5, (1962), pp. 25-29. In the examples, percentages are all by weight/volume.

### Example 1

Monoacetoacetin was dissolved in water for injection with stirring to a concentration of 150.0 g/liter, and the resulting aqueous monoacetoacetin solution was subjected to sterilizing filtration and packed into an infusion container. The air in the container was replaced with nitrogen gas, after which the container was sealed up, whereby a pharmaceutical composition for treating tumor of the present invention was prepared in the form of an infusion having a monoacetoacetin concentration of 15.0%.

### Example 2

Monoacetoacetin was dissolved in water for injection to a concentration of 100.0 g/liter. Thereafter, sterilizing filtration and packing into a container were conducted in the same manner as in Example 1, whereby a pharmaceutical composition for treating tumor of this invention was prepared in the form of an infusion having a monoacetoacetin concentration of 10.0%.

### Example 3

Monoacetoacetin was dissolved in water for injection to a concentration of 200 g/liter. Thereafter, in the same manner as in Example 2, a pharmaceutical composition for treating tumor of this invention was prepared in the form of an infusion having a monoacetoacetin concentration of 20.0%.

Examples 4 to 6

Monoacetoacetin was dissolved in water for injection to a concentration of 30.0 g/liter, 50.0 g/liter or 70.0 g/liter. Thereafter, each of the solutions thus obtained was subjected to sterilizing filtration and packing into a container in the same manner as in Example 1, whereby pharmaceutical compositions for treating tumor of this invention were prepared in the form of an infusion having monoacetoacetin concentrations of 3%, 5% and 7%, respectively.

Pharmacological Test Example 1

①Materials and Method

Donryu rats weighing 200 to 250 g were used. Under anesthesia condition by intraperitoneal administration of 40 mg/kg of Nembutal (pentobarbital sodium injection preparation, manufactured by Dainippon Pharmaceutics Co., Ltd.), a small-diameter silicon tube (inside diameter 0.5 mm, outside diameter 1.0 mm) was inserted into the ascending vena cava through the right jugular. The end of the catheter was taken out from the back through the subcutaneous tunnel, protected with a wire, passed through a button and a swivel, and set in an IV pump (Alimepump AP-200, Nipro). Administration was continuously carried out without restraint on the rats.

Four experimental groups of 5 rats each [group M (the present invention), group G (reference), group MP (the present invention), and group GP (reference)] were used. For these groups, the test infusions described below were employed respectively.

The volume of the test infusion administered to to the rats of each group was 60 to 75 ml per rat a day.

| Experimental group | Test infusion |
|---|---|
| Group M | 15% Monoacetoacetin solution (Example 1) |
| Group G | 15% Glucose solution |
| Group MP | 15% Monoacetoacetin solution + 8% multiple amino acid solution [Panamin S (Vuj-N formula), manufactured by Otsuka Pharmaceutical Factory, Inc.] |
| Group GP | 15% glucose solution + 8% multiple amino acid solution (manufactured by Otsuka Pharmaceutical Factory, Inc.) |

In addition, group F (28 rats) was used as a control group. Rats of this group were allowed to take rat food (F-2, manufactured by Funahashi Nohjoh Co., Ltd.) ad libitum freely.

Two days after the start of by administration of each of the above infusions through vein 1 x $10^6$ Yoshida sarcoma cells were inoculated subcutaneously in the right femoral region of rats of each group (including group F; in the case of group F, the inoculation was carried out 2 days after the start of taking food ad libitum). Seven days after the inoculation, each rat was killed by drawing blood through the abdominal aorta.

For rats of each group, there were measured the difference of the body weights before and after the above experiment (the feeding through vein or the ad libitum taking food), the cumulative nitrogen balance value during the experiment, the weight of tumor at the end of the experiment, the amount of nuclear DNA of Yoshida sarcoma cell, etc. Each measured value is expressed as mean value ± standard deviation. A test of significance on the mean value was carried out by the student's t test.

② Test results

1) Body weight change

The body weight change was calculated for each group by subtracting the body weight after the experiment from that before the experiment and dividing the remainder by the body weight before the experiment. It is shown as a percentage in Table 1.

Table 1

| Experimental group | Body weight change % |
|---|---|
| Group G | -8.2 ± 4.1 |
| Group M | -8.4 ± 0.9 |
| Group GP | -1.9 ± 3.1 |
| Group MP | -0.6 ± 6.3 |
| Group F | +12.6 ± 8.6 |

As shown in Table 1, group GP and group MP showed a smaller decrease of body weight ($P<0.05$) than did group G and group M, but group F showed an increase of body weight.

2) Cumulative nitrogen balance

The cumulative nitrogen balance (g/kg) during the experiment of each group is shown in Table 2.

Table 2

| Experimental group | Cumulative nitrogen balance (g/kg) |
|---|---|
| Group G | -3.83 ± 1.11 |
| Group M | -2.92 ± 0.87 |
| Group GP | +2.08 ± 1.91 |
| Group MP | +3.49 ± 3.47 |

As shown in Table 2, group GP and group MP showed a better cumulative nitrogen balance ($P<0.02$) than did group G and group M.

3) Serum total protein concentration and serum albumin concentration

The serum total protein concentration and the serum albumin concentration at the end of the experiment of each group are shown in Table 3.

Table 3

| Experimental group | Serum total protein concentration (g/dl) | Serum total protein concentration (g/dl) |
|---|---|---|
| Group G | 4.16 ± 0.32 | 1.90 ± 0.14 |
| Group M | 4.46 ± 0.11 | 2.40 ± 0.10 |
| Group GP | 4.62 ± 0.55 | 2.42 ± 0.38 |
| Group MP | 4.96 ± 0.27 | 2.90 ± 0.12 |

As shown in Table 3, group M showed higher values than did group G, and group MP showed higher values than did group GP. In particular, group MP showed a significant high serum albumin concentration ($p<0.001$ for the comparison between group G and group M and $p<0.05$ for the comparison between group GP and group MP).

4) Tumor weight

The tumor weight at the end of the experiment of each group is shown in Table 4.

Table 4

| Experimental group | Tumor weight (g) |
|---|---|
| Group G | 1.00 ± 0.43 |
| Group M | 0.48 ± 0.18 |
| Group GP | 1.71 ± 0.55 |
| Group MP | 0.54 ± 0.37 |
| Group F | 2.60 ± 1.92 |

As shown in Table 4, group F showed a significantly higher value ($p<0.001$) than did the other groups excepting group GP. Group G and group GP showed a significantly higher value than did group M and group MP, respectively ($p<0.05$ for the comparison between group G and group M and $p<0.01$ for the comparison between group GP and group MP).

5) Amount of tumor cell nuclear DNA

The over-4c rate calculated from the amount of tumor cell nuclear DNA is shown in Table 5.

Table 5

| Experimental group | Over-4c rate |
|---|---|
| Group G | 0.252 ± 0.036 |
| Group M | 0.118 ± 0.033 |
| Group GP | 0.378 ± 0.033 |
| Group MP | 0.272 ± 0.011 |
| Group F | 0.414 ± 0.069 |

As shown in Table 5, group F showed a significantly higher value ($p<0.01$) than did the other groups excepting group GP. Group GP showed a higher value ($p<0.01$) than did group g, group M and group MP.

6) Metastasis of tumor cells to other organs

In the case of group G, group M and group MP, no metastasis of tumor cells to other organs was observed.

In the case of group GP, metastasis to liver occurred in two rats (40%) and metastasis to lymph node in three rats (60%).

In the case of group F, metastasis to liver occurred in 12 rats (43%), metastasis to lung in 6 rats (21%), and metastasis to lymph node in all the rats.

From the experiment results described above, the following are clear.

① As demonstrated by taking the case of group M, monoacetoacetin is by no means nutritionally inferior as energy source to glucose (group G).

② Monoacetoacetin can inhibit the multiplication of the tumor while maintaining the nutritive conditions of an animal and does not make the tumor bearing animal starving or ill-fed. Therefore, it can

bring about a life-prolonging effect on the tumor bearing animal without causing a lowering of the immunological ability, cachexia, etc.

③ As is clear from the comparison between group M and group G, group M showed a significant decrease in tumor weight and an increase in tumor cells which were found to be dipioids by measuring the amount of nuclear DNA of the tumor cells. Therefore, we conjecture that the tumor multiplication inhibiting effect of mono-acetoacetin is not a mere nutritional effect but an effect of promoting the differentiation of tumorous cells positively to inhibit their multiplication and reduce their metastasis.

From these facts, it is concluded that the pharmaceutical composition for treating tumor of this invention is very effective in treating malignant tumor.

## Claims

1.Use of any one or both monoacetoacetins of the formulas (1) and (2),

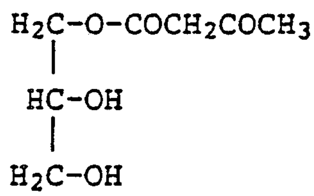

(1)

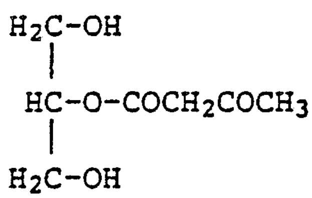

(2)

for preparing a pharmaceutical composition for treating tumor.

2. A pharmaceutical composition for treating tumor containing, as the active ingredient, any one or both monoacetoacetins of the formulas (1) and (2),

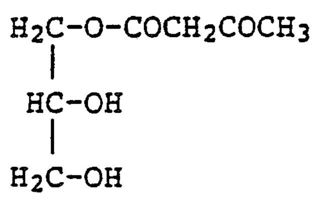

(1)

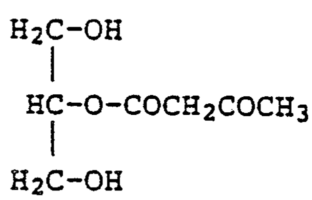

(2)

and at least one pharmaceutically acceptable carrier.